Europäisches Patentamt

European Patent Office

Office européen des brevets

(11) Publication number: **0 329 815 B1**

# EUROPEAN PATENT SPECIFICATION

(45) Date of publication of patent specification: **12.08.92**　(51) Int. Cl.⁵: **A61F 13/06**, A63B 71/12

(21) Application number: **88106757.3**

(22) Date of filing: **27.04.88**

(54) **A patella brace.**

(30) Priority: **22.02.88 JP 39112/88**

(43) Date of publication of application:
**30.08.89 Bulletin 89/35**

(45) Publication of the grant of the patent:
**12.08.92 Bulletin 92/33**

(84) Designated Contracting States:
**BE DE FR GB IT NL SE**

(56) References cited:
**EP-A- 0 115 029**
**JP-A-60 262 255**
**US-A- 4 466 428**

(73) Proprietor: **NAKAMURA BRACE CO., LTD.**
**132, Omori-cho-ha**
**Oda-shi Shimane(JP)**

(72) Inventor: **Watanabe, Tetsuya**
**2408, Sakka Kute-cho**
**Oda-shi,Shimane(JP)**
Inventor: **Nakamura, Toshiro**
**132, Omori-cho-ha**
**Oda-shi,Shimane(JP)**

(74) Representative: **Dipl.-Ing. H. Hauck Dipl.-Ing. E. Graalfs Dipl.-Ing. W. Wehnert Dr.-Ing. W. Döring**
**Neuer Wall 41**
**W-2000 Hamburg 36(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

The present invention relates to a patella brace used for brace therapy of knee joint trouble, particularly moving patella, trouble according to the preamble of the claim. More particularly, it relates to the gadget for preventing the troubles of kneejoints in sports such as volleyball and basket ball in which kneejoints usually undergo excessive physical burdens.

Troubles of patella/thighbone should be treated in accordance with the individual conditions of the patients, that is, the type and degree of the trouble. Operation of a patient in serious trouble has been thoroughly studied and is being well attended, while the preservative therapy of less serious trouble, particularly the brace therapy has not been studied satisfactory without any method to be particularly mentioned.

It is because of the drawbacks of the conventional patella brace which restrict the user's mobility, giving great inconveniences in daily life and causing functional troubles to other parts of the body, and that its wearing is especially disliked by women for aesthetic reasons.

Many girls participate in the sports club activities such as volleyball and basketball in these days in junior and senior high schools. Some of them are suffering from patella trouble. The symptom in most of these cases is that the patella moves laterally outwards (toward the gastrocnemius side or a little upward). The cause of occurrence and reason why it concentrates on girls are not yet clear, and any effective therapy is not yet known. The only method usually used is to wear a therapeutical gadget for correcting the dislocation of patella. Such a gadget is, however, generally bulky, difficult to handle, with a sense of incompatible, and disagreeable in appearance.

The knee joint is situated between the bottom end of the thighbone and the top end of the shinebone behind the patella. These bones are jointed together by many ligaments such as the joint capsule, inside and outside accessory ligaments, and cruciate ligament in the joint cavity, and are limited in direction and range of motion by these ligaments.

Since the reinforcement by these muscles are very powerful (this is readily proven by the fact that a fracture of bone is more apt to occur than a dislocation of joint by an external injury), the balance in reinforcement strength may be badly broken, affecting directly other parts when a trouble occurs. A point the most vulnerable to it is the patella. When a therapy is to be applied to cure a subluxation or fracture of patella by putting on a gadget, all bones and muscles concerned are moved or varied in position in complicated manner as the knee is bent or stretched. Thus, it is difficult to fix these parts and almost impossible to make therapy by mere forced pulling.

The conventional knee braces are roughly classified into two types. Among them the fixing-by-winding type fixes the upper and lower parts of the knee joint, that is, the lower part of the thigh and the upper part of the crus and confines the patella in position by strong fixing force.

The cylindrical type is composed of an elastic fabric having a sufficient stretchability to allow the foot and the heel to pass through when it is put on or taken off. It is rather superior in giving mobility to the body than the general fixing-by-winding type and is widely used as knee supporters for sporting.

In any case, the conventional gadgets are generally large in size except those supporters for sports use which are hard to expect to be effective in the therapy use. The fixing-by-winding type, in particular, which must be firmly secured in the upper and lower portions of the knee joint is conspicuous in this trend. It is not suitable as a gadget for therapy in view of the fact that the gadget therapy requires a long period than others such as the operational therapy. The cylindrical type, despite its drawback that the correcting effect is smaller, it is certainly suitable for sportsmen who move vigorously, because it is difficult to drop off once put on. However, for patients of patella, particularly for those who does not need to be helped by persons in daily behaviors, the cylindrical gadget is very inconvenient, because it is necessary for putting on the gadget to bend the body or the leg deeply. Furthermore, it is to decrease the pressure of the cylindrical brace on the patella gradually as the curing therapy proceeds.

In consideration of these points, the inventors designed previously a brace which had the advantages of fixing-by-winding type and cylindrical type together and covered up the drawbacks of both the types.

This device comprises a fixing member with an opening for exposing the patella and connecting belts provided on both the ends of fixing member to be connected with each other with a stretch for applying an adequate pressure on the patella. The fixing part comprises a fixing means of silicone rubber inserted between covering cloths having a plane, flexible configuration to fit to the knee joint.

This brace has various advantages such as it is very easy in attaching as well as detaching, giving scarcely a feel of incompatibility while wearing, compact in size and light in weight, accompanying no nasty feel due to perspiration, and that the pressure on the affected part can be adjusted as required according to the stage of progress of therapy. However, a number of problems were found by the studies and test uses thereafter. They are: (1) when the brace is applied to the affected part, with the expansion of the fixing member for exposing the patella, a dislocation of the opening takes place from the knee joint resulting in decreased pressure on the patella, and (2) the brace cannot cope with a minute difference in the direction of pressure to be applied from a patient to another. Against these problems, remedies had to be taken especially with regard to (1) for fundamental improvement, see for example JP-A-62/20850.

This patella brace is provided with a fixing member pressing band on the surface side of the patella fixing member in the form of covering the opening in the same direction as the longitudinal direction of the connecting belt, and with a patella pressing patch on the back of the fixing member pressing band. In short, the patella fixing member is deformed to fit to the shape of the knee joint when the brace is worn, and the fixing member pressing band presses down the patella fixing member so that the opening in the fixing member does not separate from the knee joint. Further, the patella pressing pad defines the direction of the pressure given to the patella.

The fixing member pressing band, however, is for preventing decrease of the patella holding power due to extended opening when the brace is worn, and is not so powerful. Because, if this is made powerful, the band presses the patella too strongly, hindering the bending and stretching of the knee, and lowering the reinforcement by the patella pressing patch.

Thus, although the brace have a large correcting effect, the opening continues to undergo deformation responding to the stretching of the knee joint. If a person such as skier who repeats vigorous bending and stretching of the knee wears the brace for preventing knee joint trouble, the fixing part is apt to break around the opening.

However, silicone rubber has characteristics best suited as the material of fixing part, and cannot be substituted by other material. Therefore, the brace have to be a rather short life product. (This is, of course, the case of trouble prevention for sports players who make vigorous bending and stretching of the knee joint, and is hardly a problem for ordinary preservative therapy.)

The patella brace according to the invention comprises a patella fixing member made of silicone rubber enclosing a stretchable knit or woven fabric over the whole area. Accordingly the patella fixing member is suitably reinforced and is less subjected to tearing.

Figure 1 is a perspective view showing an embodiment of the patella brace according to the invention,

Figure 2 is a schematic section of the internal structure of the patella fixing part according to the invention,

Figure 3 is a perspective view viewed from the back side to show another embodiment of the invention,

Figure 4 is a perspective view showing a condition of the patella brace of Fig. 1 worn on the left foot,

Figure 5 is a plan view showing another embodiment of the patella fixing part of the patella brace with belt presser according to the invention, and

Figures 6(a) and (b) show an embodiment of the patella brace pressing patch, Fig. 6(a) being a perspective view and Fig. 6(b) being a plan view.

DETAILED DESCRIPTION OF THE INVENTION

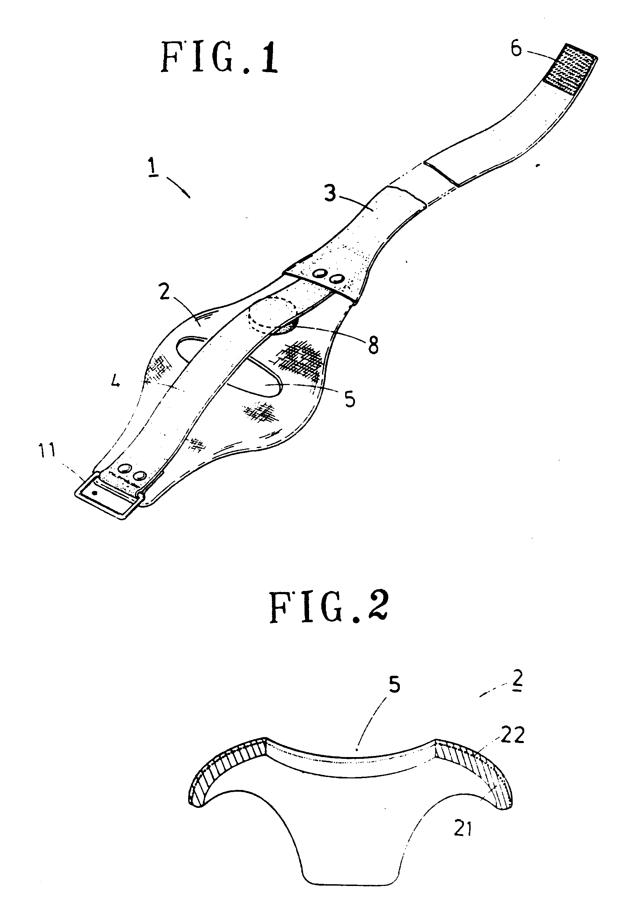

As obvious from Fig. 1, the patella brace 1 according to the invention is small-sized and very light in weight, comprising a patella fixing member 2 made of silicone rubber, a connecting belt 3 attached to one end of the patella fixing member 2, and a fixing member pressing band 4 stretched over the surface side of the patella fixing member. Near the center of the patella brace 2 an elliptical opening 5 is provided.

To put on this brace, apply the opening 5 to the patella, wind the connecting belt 3 around the rear of the leg, and after passing its end through a retaining ring 11, let the plane zipper 6 on the end of the connecting belt 3 be engaged with the connecting belt 3, while stretching the belt so that an adequate pressure is applied on the affected part.

The patella fixing member 2 is composed of silicone rubber 11 containing knit or woven fabric 22.

In this embodiment, the knit or woven fabric 22 is spandex elastic fabric composed of polyurethane fiber. This polyurethane fiber has advantages:

① High stretchability, higher than natural rubber, not broken at an elongation by five times.

② Small sagging, excellent restoring property, with little change in natural length after repeated extension.

③ Durability. Higher than natural rubber in tensile strength. Resistant to degeneration by oxidation.

④ Readily washable. High durability against repeated washing and dry-cleaning.

⑤ Excellent uniformity.

It is suitable as an elastic fabric to be enclosed in the patella fixing member 2 of the brace according to the invention. This invention, however, is not limited to this material; another material may also be used.

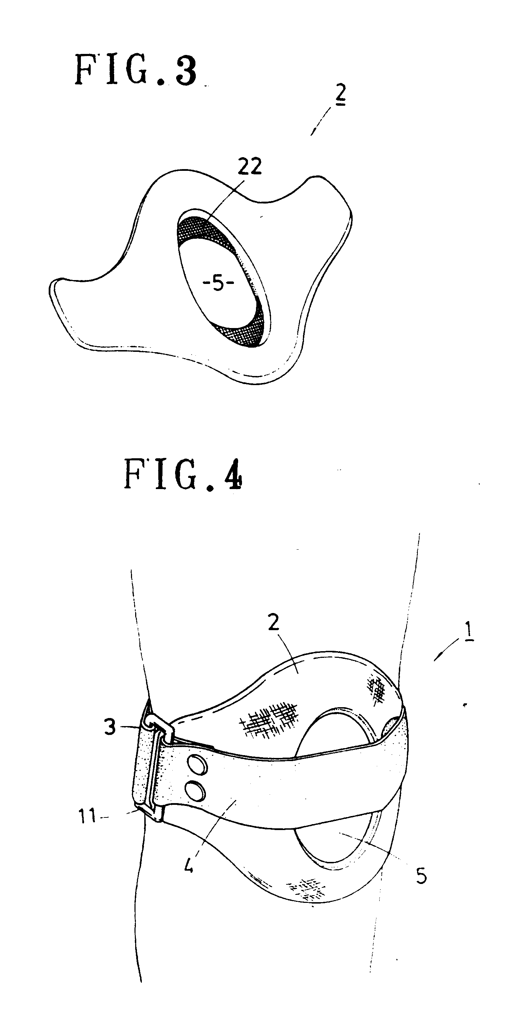

In the embodiment illustrated so far, the size of the opening 5 is entirely equal to that of the knit or woven fabric 22 layer part and silicone rubber 21 part. However, if the knit or woven fabric 22 is put on the surface side and a difference in level of the partly knit or woven fabric 22 layer is provided with the pointed part of the opening 5 as shown in Fig. 3, the resistance of the brace against a crack will increase further. Since the knit or woven fabric 22 layer part is present on the surface side of the brace, it does not affect the deformation by the stretching of the knee joint. As the method of providing a difference in level in the opening 5 as shown, available are a method of preparing in advance a patella fixing part 2 having a proper opening 5 without a knit or woven fabric 22 and joining to it a knit or woven fabric 22 with a small opening, or a method preparing a patella fixing means 2 containing a knit or woven fabric 22 with a small opening and cutting off only the silicone rubber part from the back side to form a proper opening 5. ("Proper opening" mentioned here means an opening set so as to follow the shape of the patella in generally round form when the brace is worn. "Small opening" means an opening in the kit or woven fabric having smaller major axis length than the proper opening.)

Figure 4 shows a condition of the patella brace of Fig. 1 worn on the left foot.

Tightening the connecting belt 3 applies uniform pressure on the knee joint and fixes the brace on the knee joint. Particularly, the characteristics of the silicone rubber allows little slippage of the brace. The opening 5 assumes elliptical shape in natural state, but becomes round near to the shape of the patella when worn due to the deformation by the stretching of the connecting belt 3. Here, the fixing member pressing band 4 provided on the surface of the patella fixing member 2 is stretched accompanying the deformation of the fixing member 2 and presses down the patella fixing member 2 toward the patella. (Assuming this fixing member pressing band 4 does not exist, the opening 5 will be deformed when the knee joint is bent, decreasing wearing effect of the brace.)

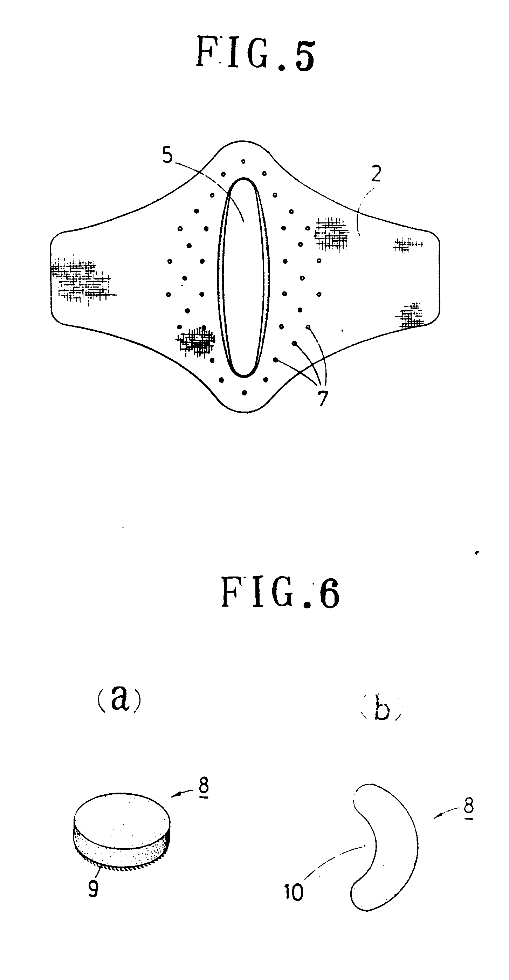

The use of silicone rubber as the material of the patella fixing member 2 brings another advantage of hygroscopicity of some degree, in addition to the loss of slippage between the skin and the high patella holding property. However, if a stuffiness by perspiration is to be feared, provision of many perforation may be made as shown in Fig. 5.

On the back of the fixing member pressing band 4, a patella pressing patch 8 is provided. This is a foamed plastics provided with a plane zipper on one face and can be attached to an adequate position on the back of the fixing member pressing band 4. The shape of the patella pressing patch 8 may be cylindrical as shown in Fig. 6(a), but may be provided with a cavity 10 as shown in Fig. 6(b) to heighten the patella holding property.

As detailed above, the patella brace according to the invention is small-sized and light weighing and is easily attachable and detachable. It gives on feeling of incompatibility even with a long period of wearing and the pressure applied in the direction of correcting dislocation is not impaired by the bending and extending of the knee. The patella fixing member made of silicone rubber develops good compatibility with the skin and the stretchable knit or woven fabric enclosed in the silicone rubber prevents production of cracks even in severe use. The product of the invention is thus small-sized and has high effect of protection, pain removal, and correction, and is used widely also as the supporter for sports.

## Claims

1. A patella brace (1) used for brace therapy of knee joint trouble, particularly moving patella trouble, comprising an elastic patella fixing member (2) having an opening (5) for exposing the patella and a connecting belt (3) to be wound round the knee joint to fix said patella fixing member (2) to the knee joint, said patella fixing member (2) being provided with a fixing member pressing band (4) on the surface of the patella fixing member in the longitudinal direction of the connecting belt, and said fixing member pressing band being provided with a patella pressing patch (8) on the back, wherein said patella fixing member (2) has a curved back and the opening (5) provided in the center of the patella fixing member (2) has an elliptical shape with minor axis in the longitudinal direction in the natural state and is made nearly round by the elongation when the brace is worn, and is made of silicone rubber, characterised in that the silicone rubber encloses a stretchable knit or woven fabric (22) over the whole area.

## Revendications

1. Bandage rotulien (1) utilisé pour soigner des troubles dans l'articulation du genou, en particulier touchant à la mobilité de la rotule,

comprenant un organe élastique (2) de maintien de rotule, présentant une ouverture (5) pour dégager la rotule, et une sangle de liaison (3) destinée à être enroulée autour de l'articulation du genou pour immobiliser ledit organe (2) sur le genou,

ledit organe (2) de maintien de rotule étant pourvu d'une bande de pressage (4) dudit organe disposée sur la surface de ce dernier dans la direction longitudinale de la sangle,

ladite bande étant équipée sur sa face arrière d'un coussinet (8) de pressage de la rotule,

bandage dans lequel ledit organe (2) de maintien de rotule présente une face arrière incurvée,

l'ouverture (5) prévue au milieu de l'organe (2) de maintien de rotule a la forme d'une éllipse dont le petit axe est dans la direction longitudinale, cette ouverture devenant à peu près ronde, par élongation, lorsque le bandage est porté,

et ledit organe (2) de maintien est en caoutchouc silicone, caractérisé en ce que le caoutchouc silicone englobe sur toute sa surface, une pièce de tissu (22) extensible, tricoté ou tissé.

## Patentansprüche

1. Patella-Bandage (1) zur Kniegelenkbehandlung, insbesondere für Patella-Verschiebungen, mit einem elastischen Patella-Fixierteil (2) mit einer Öffnung (5) im Bereich der Patella und einem Befestigungsgurt (3), der um das Kniegelenk zum Fixieren des Patella-Fixierteils (2) am Kniegelenk gewickelt wird, wobei das Patella-Fixierteil (2) mit einem Druckband (4) auf der Oberseite des Patella-Fixierteils in Längsrichtung des Befestigungsgurtes versehen ist und das Druckband ein Patella-Druckpolster (8) auf der Rückseite aufweist, wobei das Patella-Fixierteil (2) eine gekrümmte Rückseite besitzt, die Öffnung (5) in der Mitte des Patella-Fixierteils (2) elliptisch ist, die kürzere Achse der Öffnung im natürlichen Zustand in Längsrichtung verläuft und die Öffnung beim Tragen eine nahezu runde Form annimmt, und wobei das Patella-Fixierteil aus Silikongummi hergestellt ist, dadurch gekennzeichnet, daß der Silikongummi eine dehnbare gestrickte oder gewebte Einlage (22) im gesamten Bereich einschließt.

# FIG.1

# FIG.2

# FIG.3

# FIG.4

# FIG.5

# FIG.6

(a)

(b)